# FASCICULE DE BREVET EUROPEEN

(11) **EP 1 871 390 B1**
(45) Date de publication et mention de la délivrance du brevet: **25.06.2014**
(21) Numéro de dépôt: 06754744.8
(22) Date de dépôt: 14.04.2006
(51) Int. Cl.: A61K 31/7076, A61P 25/28, A23K 1/16

(54) **S-ADENOSYL-L-METHIONINE POUR LA REGULATION DES TROUBLES DU COMPORTEMENT CHEZ LES ANIMAUX DE COMPAGNIE**
S-ADENOSYL-L-METHIONIN ZUR REGELUNG VON VERHALTENSSTÖRUNGEN BEI HAUSTIEREN
S-ADENOSYL-L-METHIONINE FOR REGULATING BEHAVIORAL DISORDERS IN PETS

(30) Priorité: 15.04.2005 FR 0503782
(43) Date de publication de la demande: 02.01.2008
(73) Titulaire: VIRBAC, 06516 Carros (FR)
(72) Inventeur: BUFALA, Georges, F-06600 Antibes (FR); REME, Christophe, F-06200 Nice (FR)
(74) Mandataire: Lienard, Benoît
(86) Numéro de dépôt international: PCT/EP2006/061619
(87) Numéro de publication internationale: WO 2006/108880

(56) Documents cités:
- WO-A-01/85165
- WO-A-02/32434
- WO-A-02/45525
- WO-A-2005/006877
- FR-A- 2 823 211
- LANDSBERG G: "Therapeutic agents for the treatment of cognitive dysfunction syndrome in senior dogs" PROGRESS IN NEURO-PSYCHOPHARMACOLOGY & BIOLOGICAL PSYCHIATRY, OXFORD, GB, vol. 29, no. 3, mars 2005 (2005-03), pages 471-479, XP004809117 ISSN: 0278-5846

## Description

La présente invention concerne l'utilisation de la S-adénosyl-L-méthionine et de ses sels pour la mise en oeuvre d'une méthode pour réguler les problèmes comportementaux chez les animaux de compagnie, étant liés à l'âge et en particulier ceux associés au syndrome de dysfonctionnement cognitif (SDC). L'invention concerne aussi l'utilisation de la S-adénosyl-L-méthionine ou de ses sels pharmaceutiquement acceptables pour la préparation d'un médicament pour prévenir ou traiter le syndrome de dysfonctionnement cognitif chez les animaux de compagnie, en particulier les chiens et en particulier les chats.

Le syndrome de dysfonctionnement cognitif (SDC) est un désordre comportemental lié à l'âge. Il est observé chez les chiens et les chats et se caractérise par une détérioration des aptitudes cognitives (telles que l'orientation spatiale, le contact avec les humains, l'apprentissage, ...) et il n'est pas attribué à des dysfonctionnements moteurs ou sensoriels ou à des conditions médicales générales, telles que néoplasies, infections ou insuffisances organiques. Chez le chien, le SDC est souvent référencé par les vétérinaires comme le "syndrome du vieux chien" ou, simplement, la "sénilité". Il se manifeste par l'un au moins des cinq changements comportementaux suivants, en l'absence de causes physiques :
- Désorientation/confusion ;
- Diminution ou dégradation de l'interaction (de la réactivité) avec les membres de la famille du propriétaire ;
- Perturbation du cycle sommeil /activité ;
- Diminution du niveau d'activité ;
- Perte des habitudes de propreté ;

Des symptômes similaires peuvent être observés chez les chats et l'on parle de syndrome de dysfonctionnement cognitif félin.

La prévalence du syndrome de dysfonctionnement cognitif, chez les vieux chiens par exemple, est importante. Une étude sur le sujet a montré que 62 % des chiens âgés de 11 à 16 ans répondent positivement à une ou plusieurs des catégories comportementales caractéristiques du SDC.

Les causes du SDC ne sont pas réellement connues. Des études ont montré que les changements comportementaux associés à ce syndrome augmentent avec l'âge, et que de nombreux changements pathologiques, apparaissant chez les chiens et les chats âgés, pouvaient causer le SDC.

L'identification des changements comportementaux qui sont directement et uniquement associés au SDC chez un animal de compagnie est relativement difficile pour le praticien car elle repose sur une procédure "d'exclusion". En effet, de nombreuses maladies ont pour symptômes des changements comportementaux identiques à ceux qui sont directement associés au SDC. Ainsi, le praticien vétérinaire ne pourra conclure au SDC que s'il n'a pas diagnostiqué d'autres états pathologiques auxquels seraient associés les mêmes symptômes comportementaux.

Des exemples de maladies ne devant pas être confondues avec le SDC et auxquelles peuvent être associés certains symptômes comportementaux sont listés ci-dessous :

| Condition médicale | Changements comportementaux associés |
|---|---|
| Dysfonctionnement sensoriel (perte de la vue, de l'ouïe, de l'odorat) | Augmentation de l'irritabilité, peur ou agressivité ; diminution de l'appétit ; augmentation des vocalisations ; changements du cycle veille/sommeil ; désorientation ; diminution du comportement d'accueil ; inattentif, diminution de la réponse aux ordres verbaux |
| Maladie de l'appareil urinaire ; maladie rénale Arthrose | Incontinence, perte de la propreté, polyurie, polyphagie Faible, mobilité ou activité réduite ; irritabilité, éliminations inappropriées possible |
| Hypothyroïdie | Diminution de l'activité, augmentation de l'irritabilité et de l'agressivité |
| Dérégulation de l'axe hypophysosurrénalien (hypercorticisme) | Polyphagie, polyurie, diminution de l'interaction sociale, des réponses aux ordres et du comportement d'accueil ; réduction de l'activité ; perte de la propreté ; perturbation du cycle veille/sommeil |
| Désordres neurologiques (néoplasie intra-crânienne primaire et secondaire) | Changement du cycle veille/sommeil, des habitudes alimentaires, dans la propreté, dans l'agressivité et dans la docilité |

Quelles que soient les causes du SDC, celui-ci peut, tout comme les maladies auxquelles sont associés les mêmes symptômes comportementaux, dramatiquement affecter la santé et le bien-être d'un animal. Par conséquent, la compagnie d'un chien ou d'un chat souffrant du SDC peut rapidement devenir moins agréable, voire difficile à supporter, surtout si la sévérité des changements comportementaux associés tels que la dépression, l'anxiété, l'agressivité ou le manque de propreté augmente. Dans les cas extrêmes, l'euthanasie de l'animal peut être envisagée.

Il est difficile de fixer une durée de vie pour chaque chien mais il n'est pas rare d'en rencontrer qui portent fièrement leurs quinze ans et plus. Si l'on doit fixer une moyenne (toutes races confondues), celle-ci se situe vers les douze ans en tenant compte de tous les paramètres et notamment de la mortalité précoce (vers huit ans) due aux cancers et problèmes cardiaques. Une étude conduite en Angleterre établit que l'âge moyen au moment du décès, toutes causes confondues, varie considérablement en fonction de la race considérée. Il serait de près de 15 ans chez le caniche nain, de 13 ans chez le dalmatien, le fox terrier ou le chihuahua et de seulement un peu plus de 4 ans chez le saint-bernard (MICHELL A-R, Longevity of British breeds of dogs and its relationship with sex, size, cardiovascular variable and diseases. Action Vétérinaire, 10 mars 2000). Une certaine variabilité existe aussi entre les races de chats en ce qui concerne leur longévité. A l'heure actuelle, l'espérance de vie moyenne du félin domestique est de 14 ans et le record de 36 ans (BARETTE D. Feeding older cats and dogs. Can. Vet. J., 1990, 31, 784-785.; TAYLOR E.J., ADAMS C., NEVILLE R. Some nutritional aspects of ageing in dogs and cats. Proc. Nutr. Soc., 1995, 54, 645-656.). Un chat est généralement considéré comme âgé à partir de 9 ou 10 ans.

Etant donné l'augmentation de l'espérance de vie des chiens et des chats, à cause notamment de l'amélioration des soins vétérinaires, de la nutrition et de la prévention des morts accidentelles, les changements comportementaux liés au vieillissement chez les animaux de compagnie sont devenus un problème d'intérêt majeur. En plus des changements de comportement déjà mentionnés précédemment, on peut également citer les modifications de la prise alimentaire par l'animal, les modifications du comportement de léchage et de toilette ou encore l'augmentation de l'agressivité et de l'irritabilité. Ceci a amené la Demanderesse à rechercher des moyens de régulation des troubles du comportement chez les animaux de compagnie, mais aussi des méthodes de prévention de l'apparition des changements comportementaux, en particulier ceux associés au SDC ainsi que des médicaments de plus en plus efficaces pour prévenir et traiter les troubles du comportement, en particulier ceux liés à l'âge et plus particulièrement les changements comportementaux associés au SDC.

Ainsi, le syndrome de dysfonctionnement cognitif chez les chiens est lui-même à présent reconnu comme une maladie aux Etats-Unis par les autorités réglementaires en charge de l'enregistrement des médicaments depuis que cet organisme a accordé une indication thérapeutique à un médicament à base de chlorhydrate de sélégeline pour le contrôle des signes cliniques associés au syndrome de dysfonctionnement cognitif chez le chien. (Freedom of Information Summary, Anipryl® Tablets for use in dogs, NADA 141-080).

Par ailleurs, des recherches intensives sur le sujet ont conduit ces dernières années au dépôt de plusieurs demandes de brevet relatives à de nouvelles compositions pharmaceutiques. Le brevet EP 0 623 344, par exemple, concerne l'utilisation du chlorhydrate de sélégéline pour traiter les troubles du comportement chez le chien ou le chat. Le brevet EP 1 050 303 décrit pour sa part l'utilisation d'inhibiteur de l'acétylcholinestérase pour traiter les troubles comportementaux liés à l'âge chez les animaux de compagnie, dont le syndrome de dysfonctionnement cognitif. Par ailleurs, la demande de brevet WO 01/21178 décrit l'utilisation d'une composition contenant une quantité efficace de propentofylline pour traiter le syndrome de dysfonctionnement cognitif chez les chiens ou encore le brevet EP 0 473 252 décrit l'utilisation du composé L-déprényl pour retarder la détérioration due au vieillissement chez le chien et notamment celles des fonctions cognitives.

Le numéro spécial de 2004 (volume 35) de la revue "Le Point Vétérinaire", intitulé "les traitements du comportement du chien et du chat", fait le point sur les traitements en médecine comportementale vétérinaire en abordant toutes les facettes de la spécialité, de la chimiothérapie à la thérapie comportementale, des bases neurophysiologiques aux interventions écologiques, de la chirurgie à la phéromonothérapie. Certains articles de cette revue établissent une liste exhaustive des différents traitements biologiques actuels utilisés dans les troubles du comportement liés à l'âge. On retrouve notamment les inhibiteurs de la mono-amine-oxydase (IMAO) tels que la sélégéline, les antidépresseurs tricycliques tels que la clomipramine, les inhibiteurs sélectifs de la recapture de la sérotonine (ISRS) tels que la fluoxétine et la fluvoxamine, les morpholines telles que la trioxazine et d'autres molécules comme par exemple le piracétam, l'adrafinil, la propentofylline, la nicergoline et la vincamine.

L'article de Gary Landsberg (Therapeutic agents for the treatment of cognitive dysfunction syndrome in senior dogs, Progress in Neuro-Psychopharmacology & Biological Psychiatry 29 (2005) 471-479) énumère également un certain nombre de traitement actuellement mis en oeuvre ou envisageable contre ce syndrome.

Au cours de ses propres travaux de recherche, la Demanderesse a trouvé, de manière surprenante, que l'administration d'une composition à base de S-adénosyl-L-méthionine ou de l'un de ses sels, à un animal de compagnie présentant un ou plusieurs changements de comportement liés à l'âge, en particulier ceux associés au syndrome de dysfonctionnement cognitif, permet de réduire l'intensité de ces changements de comportement, voire de rétablir un comportement normal chez l'animal.

La S-adénosyl-L-méthionine ou 5'-[[(3S-amino-3-carboxy-propyl]-méthyl-(S)-sulfonio]-5'-déoxyadénosine, connue sous les abréviations SAM et SAMe, est un métabolite naturel de la méthionine. Cette molécule est présente dans tous les tissus des organismes vivants et intervient dans de très nombreux processus biochimiques. En particulier, elle joue le rôle de donneur de fonctions méthyles dans un nombre important de transméthylations, et de précurseur dans la synthèse de composés soufrés comme le glutathion, la cystéine, la taurine et le coenzyme A.

Dans l'organisme, le SAMe est produit à partir de L-méthionine et d'ATP par la méthionine adénosyl-transférase. Il est donc possible d'augmenter les niveaux tissulaires de SAMe en administrant de larges doses de L-méthionine exogène. Cependant, la méthionine semble être toxique à fortes doses, ce qui n'est pas le cas du SAMe. Néanmoins, le SAMe est un composé instable qui est rapidement dégradé au contact de l'air et de l'humidité, ce qui rend difficile son utilisation. Une faible quantité de SAMe est présente dans l'alimentation mais, du fait de son instabilité, cette voie d'apport de SAMe dans l'organisme est négligeable.

Il existe de nombreux ouvrages traitant des différentes actions biologiques du SAMe et plusieurs brevets d'invention décrivent le SAMe, ses activités, ses sels, leurs propriétés et leurs utilisations. A cet effet, on peut se référer en particulier aux brevets US 3 954 726, US 4 057 686, US 4 369 177, EP 0 191 133 B1, US 4 956 173, US 5 073 546, US 5 073 546, US 5 753 213, US 5 776 911, US 6 093 703, FR 2 823 211 et WO 02/32434 ainsi qu'à l'article de Friedel et al. dans DRUGS (1989), 38(3), 389-416.

On notera en particulier que le SAMe est cité parmi les antioxydants soufrés qui peuvent être utilisés dans les aliments pour animaux de compagnie décrits dans les demandes internationales WO 02/45525 et WO 2005/006877 afin d'inhiber et/ou diminuer la détérioration mentale des animaux de compagnie, éventuellement en combinaison avec d'autres antioxydants ou des acides gras oméga-3 (WO 2005/00 6877). Il n'est pas démontré ni suggéré dans ces documents que le SAMe à lui seul peut avoir des propriétés pour réguler les problèmes de comportements chez les animaux de compagnie.

Le SAMe et ses sels possèdent de nombreuses applications thérapeutiques et non thérapeutiques, mais leur utilisation pour réguler les problèmes comportementaux chez les animaux de compagnie et notamment les changements du comportement liés à l'âge, et en particulier ceux liés au syndrome de dysfonctionnement cognitif, n'a jamais été décrite ou suggérée.

Ainsi la présente invention a pour objet l'utilisation d'un sel de S-adénosyl-L-méthionine, pour la préparation d'une composition pour réguler les problèmes comportementaux chez les animaux de compagnie telle que définie dans le jeu de revendications annexé préférentiellement chez les chiens et encore plus préférentiellement chez les chats.

De manière préférée, la composition est un médicament ou un complément nutritionnel.

De manière préférée, l'animal de compagnie est un chat.

Les problèmes comportementaux que la Demanderesse se propose de réguler sont des changements comportementaux liés à l'âge ou associés au syndrome de dysfonctionnement cognitif.

Selon un autre aspect, l'invention a également pour objet un sel de S-adénosyl-L-méthionine pour une utilisation telle que définie dans le jeu de revendications annexé.

Les changements de comportement liés à l'âge mentionnés ci-dessus sont des changements de comportement qui ne sont pas liés à une pathologie particulière, tels que notamment la désorientation/confusion; la diminution ou dégradation de l'interaction (de la réactivité) avec les membres de la famille du propriétaire; la perturbation du cycle sommeil/activité; la diminution du niveau d'activité; la perte des habitudes de propreté.

La méthode de traitement décrite peut être utilisée pour traiter n'importe quel animal de compagnie âgé, en particulier les chiens et les chats et notamment les chiens et chats seniors.

Les sels de la S-adénosyl-L-méthionine qui conviennent aux fins de la présente invention sont les sels de SAMe avec l'acide p-toluènesulfonique, et l'acide l'acide 1,4-butanedisulfonique.

Conformément à l'invention, la composition est, de préférence, adaptée à une administration par voie entérale et en particulier par voie orale, auquel cas elle peut se présenter sous la forme de comprimé sécable ou non, de tablette à croquer sécable ou non, de gélule, de boulette, de poudre, de granulé, de solution ou de suspension buvable, ou encore de pâte. L'administration de la composition pharmaceutique par voie parentérale est aussi possible.

La composition conforme à l'invention sera préparée de façon classique avec les excipients couramment utilisés dans le domaine alimentaire et/ou vétérinaire pour obtenir de telles compositions solides, liquides ou pâteuses. De préférence, on choisira un excipient approprié pour garantir la stabilité de SAMe, par exemple l'huile de paraffine, l'huile de vaseline, la cellulose microcristalline ou le stéarate de magnésium.

Conformément à l'invention, la composition peut comprendre en plus une ou plusieurs substances choisies par exemple parmi les inhibiteurs de radicaux libres, les anti-inflammatoires, les acides aminés, les oligo-éléments sous forme organique ou inorganique, les vitamines et les nutriments, cette liste n'étant pas limitative. Avantageusement, on utilise le magnésium et les vitamines du groupe B.

Le médicament conforme à l'invention comprend préférentiellement un ou plusieurs principes actifs supplémentaires susceptibles d'exacerber et/ou de compléter l'activité du sel de S-adénosyl-L-méthionine dans son indication contre le syndrome de dysfonctionnement cognitif chez les animaux de compagnie. A titre de principe actif supplémentaire on peut citer par exemple le chlorhydrate de sélégiline, la propentofylline, la pentoxyfilline ou le L-déprényl, cette liste n'étant pas limitative.

Selon un mode de réalisation préféré de l'invention, les médicaments se présentent sous forme de comprimés, préférentiellement sous formes sécables aptes à être divisés en deux, en quatre ou plus, pour permettre un ajustement de la dose de SAMe en fonction du poids de l'animal devant être traité ou supplémenté.

Avantageusement, la composition est protégée contre l'air et l'humidité pour lui assurer une bonne conservation, soit par un pelliculage ou un enrobage, soit par le conditionnement du complément nutritionnel ou du médicament, soit encore par une combinaison des deux selon des méthodes bien connues de l'homme du métier.

Selon un autre mode de réalisation de l'invention, la composition est adaptée à l'administration d'une dose de sel de S-adénosyl-L-méthionine comprise entre environ 1 et environ 50 mg par kg de poids corporel et par jour.

De manière particulièrement préférée, cette composition est adaptée à l'administration d'une dose de sel de S-adénosyl-L-méthionine comprise entre environ 2,5 et environ 20 mg par kg de poids corporel et par jour et encore plus préférentiellement comprise entre environ 5 mg et environ 10 mg par kg de poids corporel et par jour. De manière préférée, cette composition est adaptée à l'administation d'une dose de sel de S-adénosyl-L-méthionine d'environ 7 mg par kg de poids corporel et par jour.

Selon une autre disposition préférée de l'invention, la composition est administrée une fois ou deux fois par jour, tous les jours pendant une période d'au minimum 15 jours, préférentiellement d'au minimum 8 semaines et encore plus préférentiellement d'au minimum 12 semaines, voire 16 semaines. Une variante pourra consister à administrer la composition sous une forme appropriée, par exemple une forme retard, à une fréquence allant d'une administration tous les deux jours jusqu'à une administration par semaine. La période de prise du complément nutritionnel et/ou de la composition pharmaceutique peut avantageusement être prolongée jusqu'à la fin de la vie de l'animal.

Dans la présente description, l'expression "réguler les problèmes comportementaux chez les animaux de compagnie, notamment les changements de comportements liés à l'âge et en particulier ceux associés au syndrome de dysfonctionnement cognitif " signifie indifféremment réduire la sévérité d'un ou plusieurs changements de comportement notamment ceux liés à l'âge ou associés au syndrome de dysfonctionnement cognitif ou encore prévenir ou retarder la survenue d'un ou plusieurs changements de comportement notamment ceux liés à l'âge ou associés au syndrome de dysfonctionnement cognitif.

L'expression " liés à l'âge " signifie qui découle des processus naturels non pathologiques du vieillissement. Chez les animaux de compagnie, et en particulier chez les chiens et chats, on parlera d'animal âgé ou encore de chien senior ou de chat senior dès lors que l'animal aura atteint un âge équivalent à 75% de la longévité moyenne de la race considérée.

L'expression " prévenir ou retarder les signes de désorientation ou de confusion" signifie diminuer la tendance de l'animal de compagnie à, par exemple, apparaître perdu, à errer sans but, à vocaliser sans raison, ou à regarder fixement dans le vide ou un mur.

L'expression "prévenir ou retarder la dégradation des interactions sociales" signifie augmenter ou rétablir la tendance d'un animal de compagnie à, par exemple, solliciter l'attention des membres de la famille du propriétaire, ou à accueillir les membres de la famille.

L'expression " prévenir ou retarder la perturbation du cycle sommeil/activité" signifie augmenter ou rétablir la tendance d'un animal de compagnie à dormir la nuit, diminuer ou rétablir la tendance d'un animal de compagnie à dormir durant le jour, ou diminuer la tendance d'un animal de compagnie à errer ou se déplacer à n'importe quelle heure d'une journée de 24 heures.

L'expression " prévenir ou retarder la perte des habitudes de propreté" signifie diminuer, par exemple, la fréquence avec laquelle l'animal de compagnie urine ou défèque à l'intérieur de la maison, urine ou défèque à l'intérieur de la maison devant les membres de la famille du propriétaire, ou urine ou défèque à l'intérieur juste après être sorti en promenade. Pour les animaux de compagnie qui ont l'habitude de signaler le désir de sortir, ces termes incluent l'amélioration de la fréquence avec laquelle un animal de compagnie demande à sortir.

L'expression " prévenir ou retarder la diminution du niveau d'activité" signifie augmenter ou rétablir la tendance d'un animal de compagnie à bouger, à jouer.

Par "complément nutritionnel", on désigne tout produit, se présentant sous forme de comprimé, de gélule, de poudre, de solution buvable, de suspension buvable, de pâte ou gel pour une administration orale ou d'autres formes médicinales habituellement non associés à des aliments, destinés à être ingérés en complément de l'alimentation courante, afin de pallier l'insuffisance réelle ou supposée des apports journaliers.

L'invention sera mieux comprise à la lecture de la description qui suit et qui illustre les propriétés inattendues des sels de SAMe, en particulier au travers d'exemples d'utilisation de compositions à base de sel de SAMe pour la mise en oeuvre de l'invention.

Il doit être bien entendu, toutefois que ces exemples ne sont donnés qu'à titre d'illustration de l'invention et n'en constituent en aucune manière une limitation.
La figure 1 illustre l'évolution du niveau d'activité moyen de chiens recevant quotidiennement la SAMe pendant 4 mois. Les scores suivants sont appliqués aux animaux: -1= niveau d'activité diminué ou visiblement diminué, 0= niveau d'activité inchangé, 1= niveau d'activité augmenté.
La figure 2 illustre l'effet de l'administration quotidiennement de la SAMe pendant 4 mois sur la durée du sommeil des chiens. Les scores suivants sont appliqués aux animaux : 0= durée du sommeil inchangée, 2= durée du sommeil augmentée ou diminuée.
La figure 3 illustre l'effet de l'administration quotidiennement de la SAMe pendant 4 mois sur l'anxiété des chiens. Les scores suivants sont appliqués aux animaux. Comportement anxieux : 0= aucun, 1= sporadique, 2= fréquent, 3= permanent.
La figure 4 illustre le score global de peur chez le chien qui est la somme des scores des 6 paramètres suivants : dilatation pupillaire, tremblements, exploration, contact avec l'homme, humeur, Bave / urine / défécation.
La figure 5 illustre l'effet de l'administration quotidiennement de la SAMe pendant 3 mois sur le cycle veille/sommeil chez les chats.
La figure 6 illustre l'effet de l'administration quotidiennement de la SAMe pendant 3 mois sur le comportement d'élimination des chats, les épisodes de malpropreté étant enregistrés.
La figure 7 illustre l'effet de l'administration quotidiennement de la SAMe pendant 3 mois sur le comportement exploratoire du chat.
La figure 8 illustre l'effet de l'administration quotidiennement de la SAMe pendant 3 mois sur l'activité de jeu du chat.
La figure 9 illustre l'effet de l'administration quotidiennement de la SAMe pendant 3 mois sur les interactions du chat avec les propriétaires ou les autres animaux. En particulier, la reconnaissance par le chat des propriétaires ou des autres animaux connus est évaluée.
La figure 10 illustre l'effet de l'administration quotidiennement de la SAMe pendant 3 mois sur l'agressivité et/ou l'irritabilité du chat qui se manifeste par exemple, quand elle existe, par les signes suivants : les pupilles sont dilatées, la bouche grande ouverte, le chat souffle, crache et montre ses canines.
La figure 11 illustre la réduction du « score de comportement gériatrique standardisé » (somme des scores sur une échelle de 1 à 3 de plusieurs troubles comportementaux) chez des chiens.

### EXEMPLE 1 : Ne fait pas partie de l'invention

### Matériel et méthode :

Une étude a été menée pour montrer l'efficacité de la S-adénosyl-L-méthionine chez des chiens âgés présentant des troubles comportementaux associés au SDC.

La S-adénosyl-L-méthionine a été administrée sous la forme de comprimés contenant 10, 50 et 100 mg de SAMe.

15 chiens âgés de plus de 7 ans ont été sélectionnés sans restriction de race, de sexe et de poids. Les chiens présentaient tous des troubles cognitifs associés au SDC (problèmes de propreté, troubles du cycle activité/sommeil, baisse d'activité) bien établis et constatés par leurs propriétaires et par des vétérinaires.

Les chiens présentant des maladies nécessitant un traitement médical ont été exclus de l'étude, de même que les chiens qui ont été traités dans le mois précédent l'étude avec des médicaments psychotropes. Par ailleurs, afin d'éviter les biais dans les observations, les traitements cardiaques, rénaux, pour le foie ainsi que les médicaments psychoactifs n'ont pas été autorisés durant la durée de l'étude.

Durant l'étude, les chiens ont reçu au minimum l'équivalent de 2,5 mg de SAMe par kg de poids corporel et par jour en une seule prise quotidienne pendant une période de 4 mois.

Les chiens ont été examinés au début de l'essai puis chaque mois par un vétérinaire. Une analyse des paramètres sanguins a été réalisée lors de chaque examen par le vétérinaire. Les propriétaires ont été également interrogés pour donner leur opinion sur les évolutions observées dans le comportement de leur animal de compagnie.

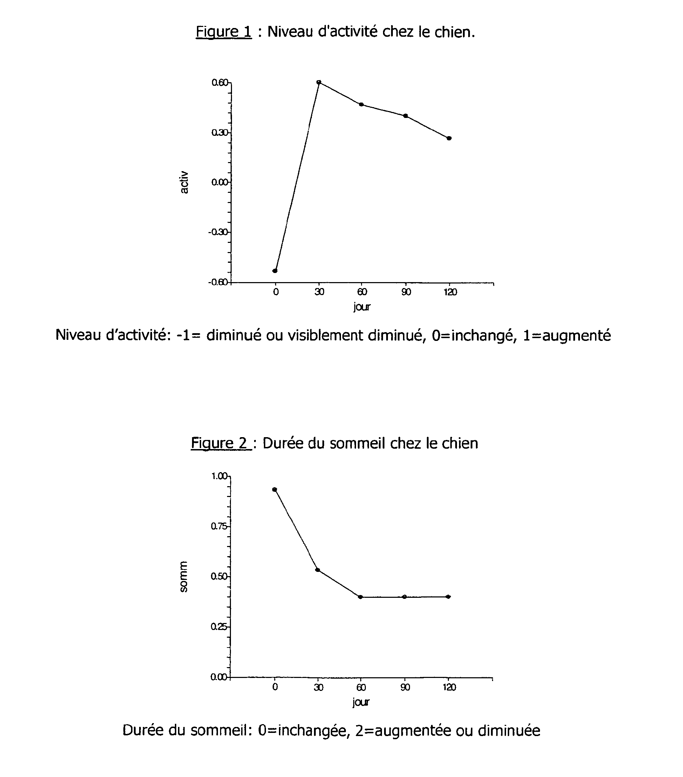

Essai numéro 1 : Mesure du niveau d'activité des chiens. Voir Figure 1.

Le niveau moyen d'activité des animaux constaté par un vétérinaire, diminué avant la prise de la SAMe, a augmenté significativement au bout d'un mois seulement. Ensuite il a diminué en moyenne mais est resté cependant globalement supérieur à l'activité normale des animaux. Les propriétaires interrogés ont pour leur part reconnu une amélioration notable dans le niveau d'activité des chiens dans les deux premières semaines du traitement (38% des chiens présente une amélioration), l'effet stimulant étant plus systématiquement reconnu après un mois (plus de 70% des chiens présentent une amélioration). La SAMe permet donc d'augmenter le niveau d'activité des animaux.

Essai numéro 2 : Mesure de l'effet sur la durée du sommeil des chiens. Voir Figure 2.

En moyenne, la durée du sommeil des animaux a eu tendance à se normaliser, les variations (augmentation ou diminution) observées en début d'essai ayant considérablement diminuées au bout de deux mois. La SAMe permet donc de réguler le cycle activité/sommeil des animaux.

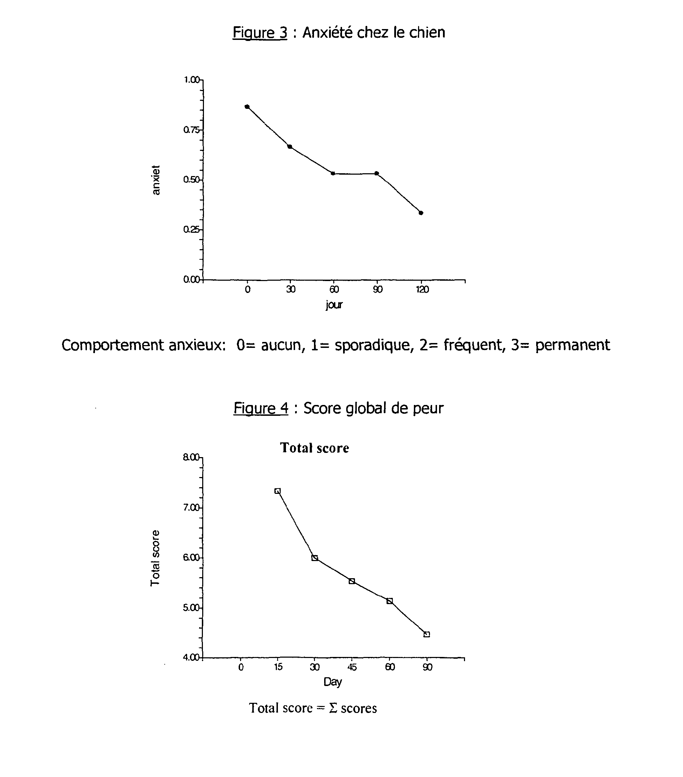

Essai numéro 3 : Evaluation de l'effet sur l'anxiété des chiens. Voir Figure 3.

L'anxiété des animaux, qui peut se manifester en particulier par un léchage excessif des pattes et du corps pouvant lui-même conduire à des dermatites dites de léchage, a diminué régulièrement pendant toute la période durant laquelle les animaux ont reçu la SAMe. La SAMe permet donc de réduire les signes d'anxiété des animaux.

### Evaluation par les propriétaires :

Tous les propriétaires des animaux étudiés ont été satisfaits par le traitement à la fin de l'étude et ont considéré l'efficacité comme très bonne. Une grande majorité des propriétaires ont remarqué une amélioration significative des paramètres particuliers et du comportement général dès la première semaine de traitement.

Les analyses biochimiques de prélèvements sanguins ont montré que la plupart des chiens avaient des valeurs normales au début de l'essai, qui sont restées dans les normes pendant toute la durée de l'essai, ce qui démontre une très bonne tolérance de la SAMe.

### Conclusion :

La SAMe administrée dans les conditions de l'essai a montré une bonne efficacité sur l'amélioration de certains troubles cognitifs chez les chiens. De plus, les propriétaires ont confirmé que le traitement a restauré quelque peu la vitalité des vieux chiens.

### EXEMPLE 2 : Ne fait pas partie de l'invention

### Matériel et méthode :

Une autre étude a été menée pour montrer l'effet de l'administration d'un complément nutritionnel à base de S-adénosyl-L-méthionine à des chiens sur le comportement et les signes physiques associés à la peur.

Le complément nutritionnel utilisé dans ce test était constitué de comprimés contenant 10, 50 et 100 mg de SAMe. Il a été administré aux animaux à une dose d'attaque de 7.5 mg de SAMe par kg de poids corporel et par jour per os en une seule prise quotidienne pendant un mois puis une dose de maintenance de 2.5 mg de SAMe par kg de poids corporel et par jour per os en une seule prise quotidienne pendant les deux mois suivants.

15 chiens âgés de 6 mois à 15 ans sont sélectionnés sans restriction de race, de sexe et de poids. Les chiens présentaient tous dans les deux mois précédant l'inclusion dans l'étude des signes de peur associés soit avec un comportement inhibé, soit au contraire avec des réponses exagérées. Ce type de problème devait avoir été identifié chez le chien depuis plus de deux mois. Le "score global de peur" (somme des scores individuels pour les différents paramètres suivis) enregistré au début de l'essai devait être supérieur à 7, les paramètres observés étant les suivants :
- Dilatation pupillaire. Scores : 0 = normal, 1 = augmentée, 2 = complète.
- Tremblements. Scores : 0 = aucun, 1 = légers, 2 = marqués.
- Exploration. Score : 0 = comportement normal, 1 = altéré (inhibé, statique), 2= très altéré (aucune exploration ou suractivité exploratoire).
- Contact avec l'homme. Score : 0 = normal, 1 = altéré (inhibé), 2 = très altéré (aucun contact ou contact permanent avec l'homme).
- Humeur. Score : 0 = calme/joie, 1 = altéré (perte de vigilance/tristesse), 2 = très altéré (excitation/déprime).
- Bave, urine, défécation. Score : 0 = non, 3 = oui.

Les chiens présentant des maladies nécessitant un traitement médical (par exemple contre des problèmes d'arthrose, rénaux, cardiaque ou tumoraux, des infections, ou des infestations) n'ont pas été inclus dans l'étude, de même que ceux ayant été traité avec des médicaments psychotropes dans le mois précédent l'étude. L'administration de médicaments psychoactifs et/ou de médicaments anti-inflammatoires stéroïdiens ou non stéroïdiens n'a pas été autorisée durant la durée de l'étude.

Les chiens ont été examinés par un investigateur au début de l'essai et toutes les deux semaines pendant deux mois ainsi qu'un mois plus tard. Les propriétaires ont également été interrogés pour donner leur opinion sur les évolutions observées dans le comportement de leur chien.

### Résultat :

On observe que chaque paramètre suivi au cours de l'essai se normalise pendant la durée de l'essai de telle sorte que le score global de peur, qui reflète le comportement des animaux et les signes physiques associés à la peur, s'il ne devient pas nul, diminue tout de même très sensiblement (voir Figure 4). Ceci reflète un effet détectable de l'administration d'un complément nutritionnel à base de S-adénosyl-L-méthionine à des chiens sur le comportement et les signes physiques associés à la peur.

### EXEMPLE 3 :

### Matériel et méthode :

Une étude a été menée pour montrer les effets du S-adénosyl-L-méthionine sur les troubles comportementaux liés à l'âge chez les chats.

La SAMe a été administrée sous la forme de comprimés contenant 90 mg de sel de S-adénosyl-L-méthionine (1,4-butanedisulfonate).

Les comprimés ont été donnés une fois par jour pendant une période de 3 mois consécutifs en respectant les doses suivantes :

Dose moyenne : 18 (±3,4) mg/kg/j ; Dose minimum : 12,9 mg/kg/j ; Dose maximum : 22,5 mg/kg/j.

L'étude a été réalisée sur 10 chats, sans restriction de race ou d'espèce, dont l'âge variait entre 12,5 et 19 ans, montrant des troubles cognitifs depuis en moyenne 6 mois. Ces troubles cognitifs ont été clairement établis et constatés par leur propriétaire et par des vétérinaires

Ces problèmes cognitifs se manifestaient de la manière suivante : baisse/perte d'intérêt pour l'environnement, en retrait, moins vif, prostration ; Déambulation ; Vocalises, miaulements nocturnes ; Durée du sommeil augmentée ; Baisse de l'activité de toilettage ; Malpropreté fécale occasionnelle.

Une analyse des paramètres sanguins a été réalisée tous les mois par le vétérinaire. Les propriétaires ont également été interrogés pour donner leur opinion sur les évolutions observées dans le comportement de leur animal de compagnie.

Dans cette étude, les termes "inchangé" ou "normal" signifie identique à ceux que présentait l'animal lorsqu'il était adulte en pleine possession de ses moyens.

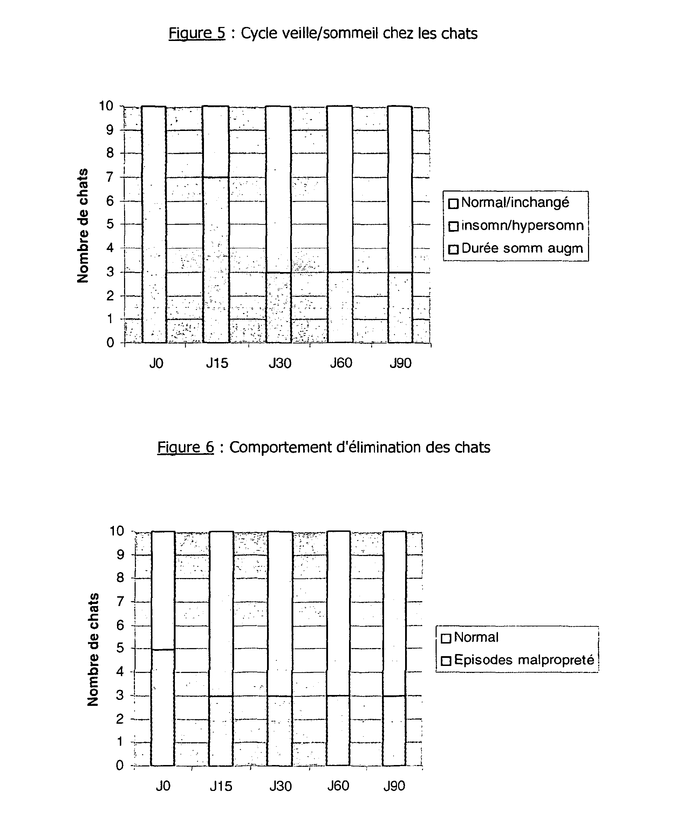

Essai numéro 1 : Mesure de l'effet du 1,4-butanedisulfonate de la SAMe sur le cycle activité/sommeil des chats. Voir Figure 5.

On a observé une reprise d'un cycle normal jour/nuit pour 7 chats sur 10 en un mois. Aucun chat n'a présenté d'épisodes de réveil en sursaut ou de cauchemars dans cette étude. Grâce à la SAMe, certains chats ont dormi beaucoup mieux alors qu'ils confondaient le jour et la nuit. Les chats ont également moins dormi dans la journée.

Essai numéro 2 : Mesure de l'effet de la SAMe sur le comportement d'élimination des chats. Voir Figure 6.

Le chat est normalement un animal très propre capable de faire dans sa litière dès l'âge de deux mois. Ce comportement peut être modifié, notamment chez le chat âgé, celui-ci pouvant déplacer le lieu de ses déjections dans des endroits généralement "moelleux" de la maison. Dans le cadre de l'essai conduit avec la SAMe selon l'invention, ce comportement a été amélioré chez 2 chats sur 5, soit dans 40% des cas.

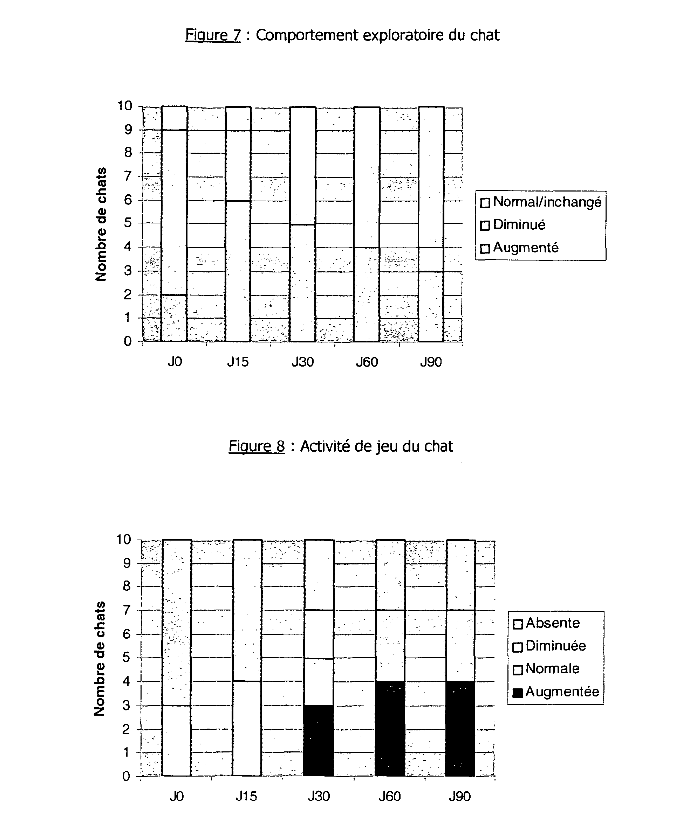

Essai numéro 3 : Mesure de l'effet de la SAMe sur le comportement exploratoire des chats. Voir Figure 7.

On a constaté que l'administration de la SAMe a un effet activateur perceptible dès 15 jours de traitement. Les chats sont plus actifs, ils miaulent pour sortir. Grâce à la SAMe, les chats sont plus actifs, plus curieux et réagissent mieux par rapport à leur environnement.

Essai numéro 4 : Comportement de déambulation ou de vocalise.

On a observé que 70% des chats présentaient des épisodes de déambulation ou de vocalise au début de l'essai alors que dès le second mois, plus aucun chat n'a montré de tels signes. L'effet est rapide puisque seulement 30 % des chats de l'essai présentaient encore des épisodes de déambulation ou de vocalise 15 jours après le début de l'essai.

Essai numéro 5 : Activité de jeu du chat. Voir Figure 8.

La SAMe a montré un net effet prometteur sur l'activité de jeu du chat à partir d'un mois. Résultat positif chez 7 chats sur 10 à 2 mois. Grâce à la SAMe, la plupart des chats ont retrouvé de l'entrain.

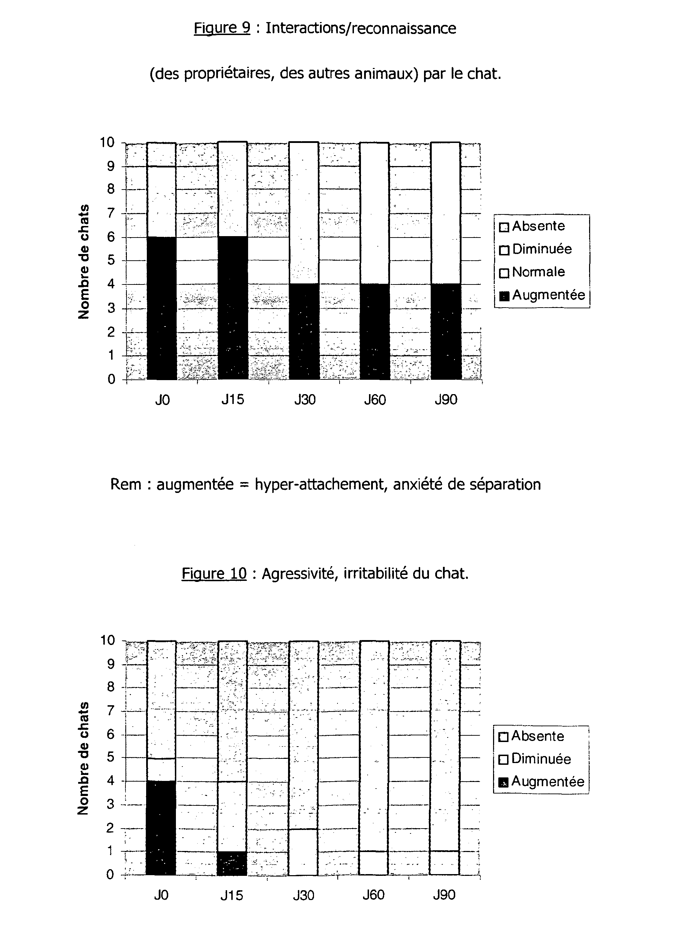

Essai numéro 6 : Interactions/reconnaissance (des propriétaires, des autres animaux). Voir Figure 9.

Les chats qui présentaient un déficit d'interaction au départ ont plutôt bien répondu (effet activateur de la composition). Les résultats sont plus variables chez les chats qui manifestaient de l'hyper-attachement.

Essai numéro 7 : Agressivité/irritabilité des chats. Voir Figure 10.

La SAMe a permis de faire disparaître en trois mois 80% (chez 4 chats sur 5) des manifestations d'agressivité ou d'irritabilité rencontrées chez 50% des chats âgés de l'essai.

### Evaluation par les propriétaires :

Les propriétaires des animaux étudiés ont été satisfaits par le traitement à la fin de l'étude et ont tous considéré l'efficacité comme très bonne. Une grande majorité des propriétaires a remarqué une amélioration significative très rapide des paramètres particuliers et du comportement général, dès la première semaine de traitement.

Les analyses biochimiques de prélèvements sanguins ont montré que la plupart des chats de l'essai avaient des valeurs normales au début de l'essai et qui sont resté dans les normes pendant toute la durée de l'essai, ce qui démontre une très bonne tolérance de la SAMe chez le chat.

### Conclusion :

Cette étude a montré qu'une supplémentation régulière des chats âgés avec la S-Adénosyl-L-Méthionine a permis d'améliorer et de rétablir un grand nombre des comportements habituels du chat sans entraîner d'effets secondaires.

### EXEMPLE 4 :

### Matériel et méthode :

Une étude randomisée en double aveugle contre placebo a été menée pour montrer les effets du S-adénosyl-L-méthionine sur la réduction de la capacité mentale chez les chiens âgés.

L'étude a été réalisée sur 36 chiens, sans restriction de race ou d'espèce, dont la moyenne d'âge est supérieure à 8 ans, montrant un ou plusieurs troubles comportementaux depuis au moins un mois. Ces troubles comportementaux ont été clairement établis et constatés par leur propriétaire et par des vétérinaires.

Ces troubles comportementaux se manifestaient de la manière suivante : désorientation, baisse de l'activité, baisse des interactions sociales, changements dans le cycle activité/sommeil, malpropreté, anxiété.

Les chiens ont été répartis au hasard dans un des deux groupes de traitement : SAMe ou placebo. La SAMe (sel de tosylate) a été administrée sous la forme de comprimés une fois par jour pendant une période d'un mois en respectant la dose moyenne de 18,5 mg/kg/j.

Les chiens on été examinés par un investigateur au début de l'essai puis à 30 jours et à 60 jours. Les propriétaires ont également été interrogés pour donner leur opinion sur les évolutions observées dans le comportement de leur chien.

Les examens ont porté sur les différents troubles comportementaux de l'animal qui ont été évalués sur une échelle de 0 à 3 en fonction de leur intensité et tous les scores ont été additionnés pour donner un « score de comportement gériatrique standardisé » reflétant la sévérité des problèmes observés.

### Résultat :

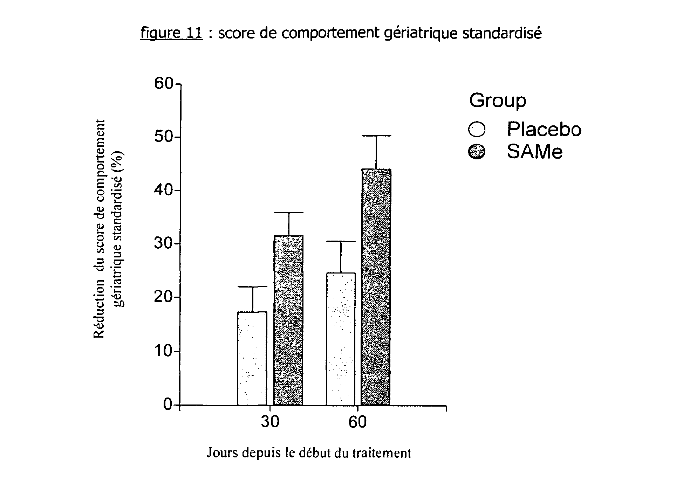

Une réduction significative du « score de comportement gériatrique standardisé » a été observée dans les deux groupes sur la période de l'étude mais la réduction était plus significative avec le SAMe qu'avec le placebo (Voir figure 11).

Plusieurs chiens dans le groupe avec le SAMe ont répondu plus favorablement au traitement que les chiens dans le groupe avec le placebo.

**Réponse au traitement à J60**

| No. (%) de chiens | Pas de réponse | Faible réponse | Bonne réponse |
|---|---|---|---|
| SAMe (n=17) | 6 (35,3%) | 4 (23,5%) | 7 (41,2%) |
| Placebo (n=19) | 12 (63,2%) | 4 (21%) | 3 (15,8%) |

| | | | |
|---|---|---|---|
| n = nombre de chiens | | | |

L'amélioration moyenne au niveau activité (57,1%), réactivité (59,5%) et comportements d'apprentissage, plus particulièrement la propreté (57,1%) était cliniquement significative après deux mois de traitement avec le SAMe.

Une majorité de propriétaires a été satisfaite par le traitement à la fin de l'étude et ils ont eu l'impression que leur animal était plus actif et réactif sous traitement.

## Revendications

1. Sel de S-adénosyl-L-méthionine pour son utilisation dans la régulation des problèmes comportementaux chez un animal de compagnie, lesdits problèmes comportementaux étant liés à l'âge mais non associés à une pathologie particulière, **caractérisé en ce que** le sel de S-adénosyl-L-méthionine est choisi parmi les sels de l'acide p-toluènesulfonique et de l'acide 1,4-butanedisulfonique, et **en ce que** ledit sel est administré à des doses journalières comprises entre 1 mg et 50 mg par kg de poids corporel, de préférence entre 2,5 et 20 mg par kg de poids corporel, et de préférence encore comprise entre 5 mg et 10 mg par kg de poids corporel.

2. Sel de S-adénosyl-L-méthionine pour une utilisation selon la revendication 1, **caractérisée en ce que** la régulation des problèmes comportementaux est la prévention ou le traitement des symptômes du syndrome de dysfonctionnement cognitif.

3. Sel de S-adénosyl-L-méthionine pour une utilisation selon la revendication 1 ou 2, **caractérisée en ce que** la quantité efficace de sel de S-adénosyl-L-méthionine est de 7 mg par kg de poids corporel et par jour.

4. Sel de S-adénosyl-L-méthionine pour une utilisation selon l'une quelconque des revendications 1 à 3, **caractérisée en ce que** le sel de S-adénosyl-L-méthionine est administrée par voie orale.

5. Sel de S-adénosyl-L-méthionine pour une utilisation selon la revendication 4, **caractérisée en ce que** le sel de S-adénosyl-L-méthionine est formulé sous l'une des formes choisies parmi les comprimés sécables ou non, les tablettes à croquer sécables ou non, les gélules, les boulettes, les poudres, les granulés, les solutions buvables, les suspensions buvables, ou les pâtes pour administration orale.

6. Sel de S-adénosyl-L-méthionine pour une utilisation selon l'une quelconque des revendications 1 à 5, **caractérisée en ce que** le sel de S-adénosyl-L-méthionine se présente sous la forme d'un complément nutritionnel ou d'un médicament.

7. Sel de S-adénosyl-L-méthionine pour une utilisation selon la revendication 6, **caractérisée en ce que** le médicament contient un ou plusieurs principes actifs supplémentaires.

8. Sel de S-adénosyl-L-méthionine pour une utilisation selon l'une quelconque des revendications 1 à 7, **caractérisée en ce qu'**une quantité efficace de sel de S-adénosyl-L-méthionine est administrée, une fois ou deux fois par jour tous les jours pendant une période d'au minimum 15 jours, de préférence d'au minimum 8 semaines, et de préférence encore d'au minimum 12 semaines, à l'animal de compagnie.

9. Utilisation d'un sel de S-adénosyl-L-méthionine pour la préparation d'une composition pour réguler les problèmes comportementaux chez un animal de compagnie, lesdits problèmes comportementaux étant liés à l'âge mais non associés à une pathologie particulière, **caractérisée en ce que** le sel de S-adénosyl-L-méthionine est choisi parmi les sels de l'acide p-toluènesulfonique et de l'acide 1,4-butanedisulfonique, et **en ce que** ledit sel est administré à des doses journalières comprises entre 1 mg et 50 mg par kg de poids corporel, de préférence entre 2,5 et 20 mg par kg de poids corporel, et de préférence encore comprise entre 5 mg et 10 mg par kg de poids corporel.

10. Utilisation selon la revendication 9, **caractérisée en ce que** la composition est un médicament pour prévenir ou traiter les symptômes du syndrome de dysfonctionnement cognitif.

11. Utilisation selon la revendication 9 ou 10, **caractérisée en ce que** la composition est un complément nutritionnel ou un médicament.

12. Utilisation selon l'une quelconque des revendications 9 à 11, **caractérisée en ce qu'**une quantité efficace de sel de S-adénosyl-L-méthionine est administrée, une fois ou deux fois par jour tous les jours pendant une période d'au minimum 15 jours, de préférence d'au minimum 8 semaines, et de préférence encore d'au minimum 12 semaines, à l'animal de compagnie.

## Patentansprüche

1. Salz von S-Adenosyl-L-Methionin für seine Verwendung bei der Regulierung von Verhaltensproblemen bei einem Haustier, wobei die Verhaltensprobleme mit dem Alter verbunden, aber nicht mit einer besonderen Pathologie assoziiert sind, **dadurch gekennzeichnet, dass** das Salz von S-Adenosyl-L-Methionin aus den Salzen der p-Toluolsulfonsäure und der 1,4-Butandisulfonsäure ausgewählt ist und dass das Salz in Tagesdosen zwischen 1 mg und 50 mg pro kg Körpergewicht, vorzugsweise zwischen 2,5 und 20 mg pro kg Körpergewicht und weiterhin bevorzugt zwischen 5 mg und 10 mg pro kg Körpergewicht verabreicht wird.

2. Salz von S-Adenosyl-L-Methionin für eine Verwendung nach Anspruch 1, **dadurch gekennzeichnet, dass** die Regulierung der Verhaltensprobleme die Prävention oder die Behandlung der Symptome des kognitiven Dysfunktionssyndroms ist.

3. Salz von S-Adenosyl-L-Methionin für eine Verwendung nach Anspruch 1 oder 2, **dadurch gekennzeichnet, dass** die wirksame Menge an Salz von S-Adenosyl-L-Methionin 7 mg pro kg Körpergewicht und pro Tag beträgt.

4. Salz von S-Adenosyl-L-Methionin für eine Verwendung nach einem der Ansprüche 1 bis 3, **dadurch gekennzeichnet, dass** das Salz von S-Adenosyl-L-Methionin auf oralem Weg verabreicht wird.

5. Salz von S-Adenosyl-L-Methionin für eine Verwendung nach Anspruch 4, **dadurch gekennzeichnet, dass** das Salz von S-Adenosyl-L-Methionin in einer der Formen, die aus teilbaren oder nicht teilbaren Tabletten, teilbaren oder nicht teilbaren Kautabletten, Kapseln, Kügelchen, Pulvern, Granulaten, Trinklösungen, Trinksuspensionen oder Pasten zur oralen Verabreichung ausgewählt sind, formuliert ist.

6. Salz von S-Adenosyl-L-Methionin für eine Verwendung nach einem der Ansprüche 1 bis 5, **dadurch gekennzeichnet, dass** das Salz von S-Adenosyl-L-Methionin in Form eines Nahrungsergänzungsmittels oder eines Medikaments vorliegt.

7. Salz von S-Adenosyl-L-Methionin für eine Verwendung nach Anspruch 6, **dadurch gekennzeichnet, dass** das Medikament einen oder mehrere zusätzliche Wirkstoffe enthält.

8. Salz von S-Adenosyl-L-Methionin für eine Verwendung nach einem der Ansprüche 1 bis 7, **dadurch gekennzeichnet, dass** eine wirksame Menge an Salz von S-Adenosyl-L-Methionin dem Haustier einmal oder zweimal pro Tag, täglich, über einen Zeitraum von mindestens 2 Wochen, vorzugsweise von mindestens 8 Wochen und weiterhin vorzugsweise von mindestens 12 Wochen verabreicht wird.

9. Verwendung eines Salzes von S-Adenosyl-L-Methionin für die Zubereitung einer Zusammensetzung zur Regulierung von Verhaltensproblemen bei einem Haustier, wobei die Verhaltensprobleme mit dem Alter verbunden, aber nicht mit einer besonderen Pathologie assoziiert sind, **dadurch gekennzeichnet, dass** das Salz von S-Adenosyl-L-Methionin aus den Salzen der p-Toluolsulfonsäure und der 1,4-Butandisulfonsäure ausgewählt ist und dass das Salz in Tagesdosen zwischen 1 mg und 50 mg pro kg Körpergewicht, vorzugsweise zwischen 2,5 und 20 mg pro kg Körpergewicht und weiterhin bevorzugt zwischen 5 mg und 10 mg pro kg Körpergewicht verabreicht wird.

10. Verwendung nach Anspruch 9, **dadurch gekennzeichnet, dass** die Zusammensetzung ein Medikament ist, um den Symptomen des kognitiven Dysfunktionssyndroms vorzubeugen oder sie zu behandeln.

11. Verwendung nach Anspruch 9 oder 10, **dadurch gekennzeichnet, dass** die Zusammensetzung ein Nahrungsergänzungsmittel oder ein Medikament ist.

12. Verwendung nach einem der Ansprüche 9 bis 11, **dadurch gekennzeichnet, dass** ein wirksame Menge an Salz von S-Adenosyl-L-Methionin dem Haustier einmal oder zweimal pro Tag, täglich, über einen Zeitraum von mindestens 2 Wochen, vorzugsweise von mindestens 8 Wochen und weiterhin vorzugsweise von mindestens 12 Wochen verabreicht wird.

## Claims

1. Salt of S-adenosyl-L-methionine for use in the regulation of behavioural problems in pets, said behaviour problems being related to age but not associated with a specific pathological condition, **characterised in that** said salt of S-adenosyl-L-methionine is chosen from the salts of p-toluenesulfonic acid and 1,4-butanedisulfonic acid, and wherein said salt is administered at daily doses of between 1 mg and 50 mg per kg of body weight, preferably between 2.5 mg and 20 mg per kg of body weight, more preferably between 5 mg and 10 mg per kg of body weight.

2. The salt of S-adenosyl-L-methionine for a use according to claim 1, **characterised in that** the regulation of behavioural problems is the prevention or treatment of the symptoms of cognitive dysfunction syndrome.

3. The salt of S-adenosyl-L-methionine for a use according to claim 1 or 2, **characterised in that** the effective amount of salt of S-adenosyl-L-methionine is 7 mg per kg of body weight per day.

4. The salt of S-adenosyl-L-methionine for a use according to any one of claims 1 to 3, **characterised in that** the salt of S-adenosyl-L-methionine is for oral administration.

5. The salt of S-adenosyl-L-methionine for a use according to claim 4, **characterised in that** the salt of S-adenosyl-L-methionine is in a form selected from those comprising divisible or indivisible tablets, divisible or indivisible chewable tablets, capsules, boluses, powders, granules, solutions to be taken orally, suspensions to be taken orally, and pastes for oral administration.

6. The salt of S-adenosyl-L-methionine for a use according to any one of claims 1 to 5, **characterised in that** the composition is a nutritional supplement or a drug.

7. The salt of S-adenosyl-L-methionine for a use according to claim 6, **characterised in that** the drug contains one or more additional active principles.

8. The salt of S-adenosyl-L-methionine for a use according to any one of claims 1 to 7, **characterised in that** an effective quantity of the salt of S-adenosyl-L-methionine is administered, once or twice per day, every day, during at least 15 days, preferably at least 8 weeks, and still preferably at least 12 weeks, to the pet.

9. Use of a salt of S-adenosyl-L-methionine for the preparation of a composition for regulation of behavioural problems in pets, said behaviour problems being related to age but not associated with a specific pathological condition, **characterised in that** said salt of S-adenosyl-L-methionine is chosen from the salts of p-toluenesulfonic acid and 1,4-butanedisulfonic acid, and wherein said salt is administered at daily doses of between 1 mg and 50 mg per kg of body weight, preferably between 2.5 mg and 20 mg per kg of body weight, more preferably between 5 mg and 10 mg per kg of body weight.

10. The use according to claim 9, **characterised in that** the composition is a drug for the prevention or treatment of the symptoms of cognitive dysfunction syndrome.

11. The use according to claim 9 or 10, **characterised in that** said composition is a nutritional supplement or a drug.

12. The use according to any one of claims 9 to 11, **characterised in that** an effective quantity of the salt of S-adenosyl-L-methionine is administered, once or twice per day, every day, during at least 15 days, preferably at least 8 weeks, and still preferably at least 12 weeks, to the pet.
